# EUROPÄISCHE PATENTANMELDUNG

(11) **EP 4 147 656 A1**
(43) Veröffentlichungstag der Anmeldung: **15.03.2023**
(21) Anmeldenummer: 21196607.2
(22) Anmeldetag: 14.09.2021
(51) Int. Cl.: A61B 18/12

(54) **GENERATOR UND VERFAHREN ZUR ERZEUGUNG EINER BEHANDLUNGSSPANNUNG**

(71) Anmelder: Erbe Elektromedizin GmbH, 72072 Tübingen (DE)
(72) Erfinder: Dornhof, Konstantin, 78187 Geisingen (DE)
(74) Vertreter: Rüger Abel Patentanwälte PartGmbB

(57) **Zusammenfassung**

Ein erfindungsgemäßer Generator (11) weist eine Anzahl von Impulsgeneratoren (G₁ bis Gₙ) auf, die von einer Steuereinrichtung (18) zeitlich flexibel einzeln angesteuert werden. Die zur Versorgung eines chirurgischen Instruments (12) erforderliche HF-Spannung wird dadurch aus Einzelimpulsen zusammengesetzt. Gleiches gilt für den an der Elektrode (14) des Instruments (12) fließenden Strom. Durch den Wegfall von Resonanzeffekten in den Generatoren und den Wegfall der Energiespeicherung in einem schwingungsfähigen System (System zweiter Ordnung) erhält der Nutzer ein erhöhtes Maß an Kontrolle über die Kurvenform der an das Instrument (12) gelieferten Spannung und des zu dem Instrument (12) fließenden Stroms.

## Beschreibung

Die Erfindung betrifft einen Generator zur Versorgung medizinischer Instrumente mit einer Behandlungsspannung und einem Behandlungsstrom sowie ein Verfahren zur Erzeugung einer solchen Behandlungsspannung und eines Behandlungsstromes.

Elektrochirurgische Instrumente, Sonden und dergleichen zur Anwendung in der Elektrochirurgie, werden typischerweise mit einem hochfrequenten Wechselstrom gespeist. Die Frequenz dieses Wechselstroms oder der angelegten Wechselspannung liegt typischerweise über 100 kHz, um neuromuskuläre Reizungen zu vermeiden. Die Leistung solcher Generatoren liegt typischerweise deutlich über 1 W und kann mehrere 100 W erreichen.

Zur Erzeugung elektrochirurgischer Spannungen im Bereich mehrerer 100 kHz werden typischerweise fremdgesteuerte Generatoren angewandt, wie sie beispielsweise der DE 10 2008 039 884 A1 zu entnehmen sind. Ein solcher Generator enthält mindestens einen Schwingkreis, der über eine aktive Transistorschaltung zur Schwingung gebracht wird und aus dem elektrochirurgische Energie transformatorisch ausgekoppelt wird. Es sind verschiedene Konzepte bekannt, um die Generatorspannung zur Erzielung verschiedener Gewebeeffekte zu modulieren.

Die Änderung der Schwingung an einem Schwingkreis unterliegt zeitlichen Einschränkungen, womit auch der Art und Weise der Modulation Schranken gesetzt sind.

Es ist Aufgabe der Erfindung, einen Generator anzugeben, der erweiterte Gestaltungsmöglichkeiten hinsichtlich der erzeugten Schwingungs- und Spannungsformen eröffnet.

Diese Aufgabe wird mit dem Generator nach Anspruch 1 sowie auch mit dem Verfahren nach Anspruch 12 gelöst:

Der erfindungsgemäße Generator umfasst eine Anzahl von Impulsgeneratoren, die jeweils einen Steuereingang und einen Impulsgeneratorausgang aufweisen. Vorzugsweise ist jeder Impulsgenerator so ausgebildet, dass er bei Empfang eines Steuerimpulses an seinen Steuereingang einen Ausgangsimpuls abgibt. Vorzugsweise sind die Ausgänge der Impulsgeneratoren mit dem Ausgang des Generators verbunden, an dem die von den Impulsgeneratoren erzeugten Ausgangsimpulse eintreffen. Eine Steuereinrichtung ist mit den Steuereingängen der Impulsgeneratoren verbunden und koordiniert deren Impulsabgabe. Auf diese Weise können an dem Ausgang des Generators Impulsfolgen gleich oder unterschiedlich starker Impulse sowie sich periodisch wiederholende Impulse oder auch Impulsfolgen mit unterschiedlichen wechselnden Zeitabständen erzeugt werden. Somit wird eine große Anzahl möglicher Spannungs- und Stromformen an dem Ausgang des Generators ermöglicht, die mit einem Parallelschwingkreis nicht oder nur mit unverhältnismäßigem Aufwand erzeugbar sind. Es lassen sich Impulsfolgen mit symmetrischer Impulsform, insbesondere aber auch Impulsfolgen mit asymmetrischer Impulsform erzeugen.

Das erfindungsgemäße Konzept verteilt die Ausgangsleistung des Generators auf mehrere Impulsgeneratoren, die somit jeweils für sich lediglich einen Teilleistungsbeitrag zu der von dem Generator insgesamt abgegebenen Leistung beitragen. Dies minimiert die Belastung der einzelnen Bauteile in den Impulsgeneratoren, insbesondere deren Leistungsschalter und verringert den Kühlleistungsbedarf. So können die Leistungsschalter als integrierte Bauelemente ausgeführt werden, beispielsweise als Transistorarrays.

Vorzugsweise sind die Impulsgeneratoren keine in sich schwingungsfähigen Systeme, d.h. sie weisen vorzugsweise keine mit dem Impulsgeneratorausgang verbundene Resonanzbauelemente, Resonanzkreise oder dergleichen auf (d.h. der Ausgangskreis wird mit einer Differentialgleichung beschrieben, deren Ordnung kleiner als 2 ist, zumindest im Hinblick auf Lösungen mit Frequenzen, die in der Größenordnung der Ausgangsfrequenz liegen). Deswegen bleiben Resonanzerscheinungen aus, weswegen die Impulsgeneratoren jeweils kontrolliert Impulse abgeben können, ohne nachzuschwingen. Bei Abgabe ihres Impulses geben die Impulsgeneratoren jeweils die gesamte gespeicherte Leistung als Ausgangsimpuls ab.

Dies gibt dem Generator eine verbesserte Kontrolle über die Spannungsform und die an das Instrument und somit biologisches Gewebe abgegebene Leistung.

Vorzugsweise sind die Impulsgeneratoren untereinander gleich ausgebildet. Sie sind somit baugleich und erzeugen gleich große Ausgangsimpulse. Durch das Timing der Impulsabgabe der einzelnen Impulsgeneratoren kann die Ausgangsimpulsfolge gestaltet werden. Z.B. können Impulsgeneratoren gleichzeitig feuern (Ausgangsimpulse abgeben), so dass sich die Ausgangimpulse der Impulsgeneratoren an dem Generatorausgang addieren. Auch können Impulsfolgen erzeugt werden, die in einem festgelegten zeitlichen Schema liegende Einzelimpulse enthalten. Es ist weiter möglich, Impulsgeneratoren vorzusehen, die jeweils unterschiedliche große Ausgangsimpulse abgeben, beispielsweise um durch verschiedene Kombinationen von Ausgangsimpulsen einzelner Impulsgeneratoren verschieden große Ausgangsimpulse am Ausgang des Generators bereitzustellen.

Die Steuereinrichtung ist vorzugsweise dazu eingerichtet, Steuerimpulse in wählbaren, verschiedenen Operationsmoden zugeordneten Schemata abzugeben. Z.B. kann ein Mode vorgesehen sein, in dem an zumindest zwei der Impulsgeneratoren zeitlich aufeinander folgend abzugeben. Auf diese Weise lässt sich eine aus Einzelimpulsen bestehende Impulsfolge am Ausgang des Generators erzeugen. Diese Steuereinrichtung kann zusätzlich oder alternativ darauf eingerichtet sein, Steuerimpulse an zumindest zwei der Impulsgeneratoren gleichzeitig abzugeben. Diese Impulsgeneratoren geben an ihren Ausgängen dann Ausgangsimpulse ebenfalls gleichzeitig ab, sodass sich diese am Ausgang des Generators summieren. Auf diese Weise können in einer Einstellung der Steuereinrichtung an dem Ausgang des Generators auch Ausgangsimpulse erzeugt werden, die größer sind als andere Ausgangsimpulse. Es können so Impulsfolgen mit Impulsen wechselnder Impulsgröße und/oder unterschiedlichen Impulsabständen erzeugt werden.

Die Steuereinrichtung kann dazu eingerichtet sein, in einer ausgewählten Betriebsart Steuerimpulse in gleichmäßigen zeitlichen Abständen abzugeben. Es entsteht dann eine gleichmäßige Ausgangsimpulsfolge am Ausgang des Generators. Zur Erzeugung bestimmter chirurgischer Effekte kann die Steuereinrichtung auch dazu eingerichtet sein, in anderen Betriebsarten Steuerimpulse nach einem oder mehreren vorgegebenen zeitlichen Mustern abzugeben. Beispielsweise kann auf eine Folge von mehreren, zum Beispiel sechs Einzelimpulsen, eine größere Pause folgen, nach der wiederum eine Folge von Einzelimpulsen abgegeben wird.

Die Impulsgeneratoren des Generators weisen jeweils zumindest einen Energiespeicher auf, der insbesondere als Induktor ausgebildet sein kann. Der Impulsgeneratorausgang ist vorzugsweise eine mit dem Induktor transformatorisch koppelnde Spule. Der Induktor ist zumindest vorzugsweise nicht Teil eines schwingungsfähigen Systems insbesondere nicht Teil eines Parallelschwingkreises oder eines Reihenschwingkreises. Der Impulsgeneratorausgang wird vorzugsweise durch eine transformatorisch mit dem Induktor koppelnde Spule gebildet. Der Impulsgenerator ist vorzugsweise als Sperrwandler ausgebildet. Dieser weist einen elektronischen Schalter auf, der den Induktor wahlweise mit einer Spannungsquelle verbindet, um die Spule mit Energie aufzuladen, und danach von der Spannungsquelle trennt, um die Energie an dem Impulsgeneratorausgang als Impuls mit hoher Spannung bereitzustellen.

Bei einer bevorzugten Ausführungsform des Generators sind die Ausgänge der Impulsgeneratoren in Reihe geschaltet und somit alle mit dem Ausgang des Generators verbunden. Die den Impulsgeneratorausgang jeweils bildenden Spulen sind dabei vorzugsweise gleichsinnig in Reihe geschaltet, sodass die von den einzelnen Impulsgeneratoren abgegebenen Impulse gleichsinnig am Generatorausgang ankommen.

Es ist aber bedarfsweise auch möglich, einen oder mehrere der Spulen gegensinnig mit den anderen Spulen in Reihe zu schalten, um an den Generatorausgang sowohl positive als auch negative Spannungsimpulse bereitstellen zu können. Auf diese Weise können nicht nur asymmetrische, sondern auch symmetrische Ausgangsimpulsfolgen an dem Ausgang des Generators erzeugt werden.

Das erfindungsgemäße Verfahren beruht auf der Erzeugung einer Folge von Stromimpulsen mittels mehrerer ausgangsseitig miteinander verbundener Impulsgeneratoren. Durch die Verwendung mehrerer Impulsgeneratoren, die jeweils dazu eingerichtet sind, bei Empfang eines Steuerimpulses lediglich einen einzigen Ausgangsimpuls abzugeben, können die von dem Generator zu erzeugende Ausgangsspannung sowie der abgegebene Strom in weiten Grenzen frei gestaltet werden, wobei keine Ein- und Ausschwingvorgänge von Schwingkreisen zu berücksichtigen sind.

Einzelheiten der Erfindung sind Ausführungsbeispielen zu entnehmen, die in der nachfolgenden Beschreibung unter Bezugnahme auf eine Zeichnung erläutert ist, die folgende Figuren enthält:
Figur 1 ein Übersichtsblockbild des erfindungsgemäßen Generators,
Figur 2 und 3 Ausgangsbeispiele von Impulsgeneratoren für den Generator nach Figur 1,
Figur 4 einen vereinfachten Schaltplan des Generators nach Figur 1 und
Figur 5 bis 10 Steuersignaldiagramme und Ausgangsspannungsdiagramme des Generators nach Figur 1 und 4 in unterschiedlichen Betriebsarten.

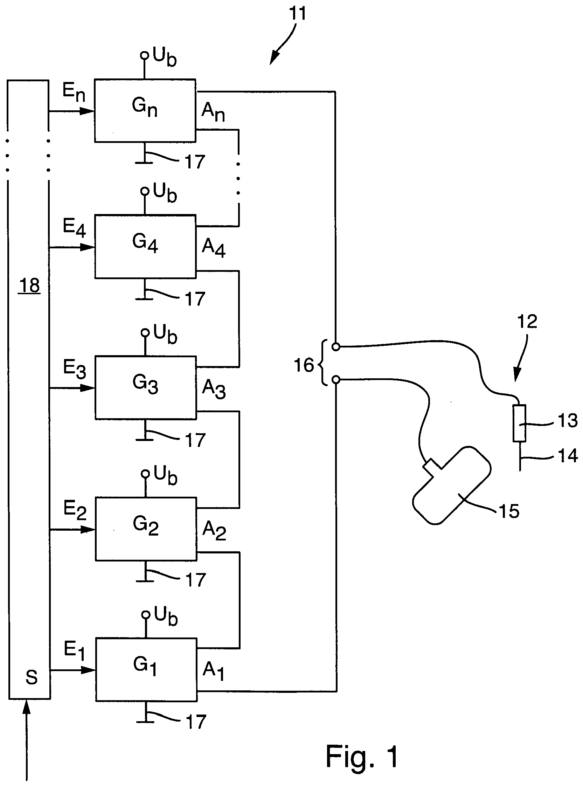

In Figur 1 ist ein Generator 11 veranschaulicht, der zur Speisung eines chirurgischen Instruments 12 dient. Als Beispiel ist in Figur 1 ein chirurgisches Instrument 12 für offenchirurgischen Einsatz mit einem Handgriff 13 und einer Elektrode 14 veranschaulicht. Es handelt sich dabei um ein monopolares Instrument. Zur Ableitung des von dem Instrument 12 auf den Patienten gehenden Stroms ist eine Neutralelektrode 15 vorgesehen, die so wie das Instrument 12 über eine geeignete Leitung an einen Ausgang 16 des Generators 11 angeschlossen ist. In Figur 1 ist beispielshalber ein monopolares Instrument 12 dargestellt. Der Generator 11 eignet sich aber ebenso gut zur Speisung bipolarer Instrumente. Außerdem kann er unabhängig davon, ob es sich um monopolare oder bipolare Instrumente handelt, zur Speisung laparoskopischer oder auch endoskopisch zu nutzender Instrumente (Sonden) vorgesehen sein.

Der Generator 11 umfasst eine Anzahl von Impulsgeneratoren G₁, G₂, G₃, G₄ ... Gₙ in geeigneter Anzahl, beispielsweise 4, 6, 12 oder auch in einer davon abweichenden Anzahl. Die Impulsgeneratoren G₁ bis Gₙ sind untereinander vorzugsweise gleich aufgebaut und parallel zueinander an eine Betriebsspannung U_{b} sowie an Masse 17 angeschlossen. Prinzipiell ist dies aber nicht zwingend; die Impulsgeneratoren G₁ bis Gₙ können auch hinsichtlich ihrer Struktur und/oder Bemessung unterschiedlich ausgebildet sein, z.B. um unterschiedlich große Ausgangsimpulse abzugeben.

Jeder Impulsgenerator G₁ bis Gₙ weist einen Impulsgeneratorausgang A₁ bis Aₙ auf, der jeweils mit dem Ausgang 16 des Generators 11 verbunden ist. Dazu sind die Impulsgeneratorausgänge A₁ bis Aₙ beispielsweise wie in Figur 1 veranschaulicht, miteinander in Reihe geschaltet. Dies ist insbesondere möglich, weil die Impulsgeneratorausgänge A₁ bis Aₙ niederohmig, d.h. innerhalb der Reihenschaltung für Ausgangsimpulse anderer Impulsgeneratoren durchlässig sind. Sollten die Impulsgeneratorausgänge A₁ bis Aₙ hochohmig sein, können sie parallel zueinander geschaltet und mit dem Ausgang 16 verbunden sein.

Weiter weist der Generator 11 eine Steuereinrichtung 18 Steuersignalausgängen auf, die mit Steuersignaleingängen E₁ bis Eₙ der Impulsgeneratoren G₁ bis Gₙ verbunden sind. Die Steuereinrichtung 18 weist ihrerseits einen Steuereingang S auf, über die Steuereinrichtung 18 ein Aktivierungssignal erhalten kann, das von einem Bedienelement, beispielsweise einem Fußschalter, einem Handschalter oder dergleichen herrühren kann.

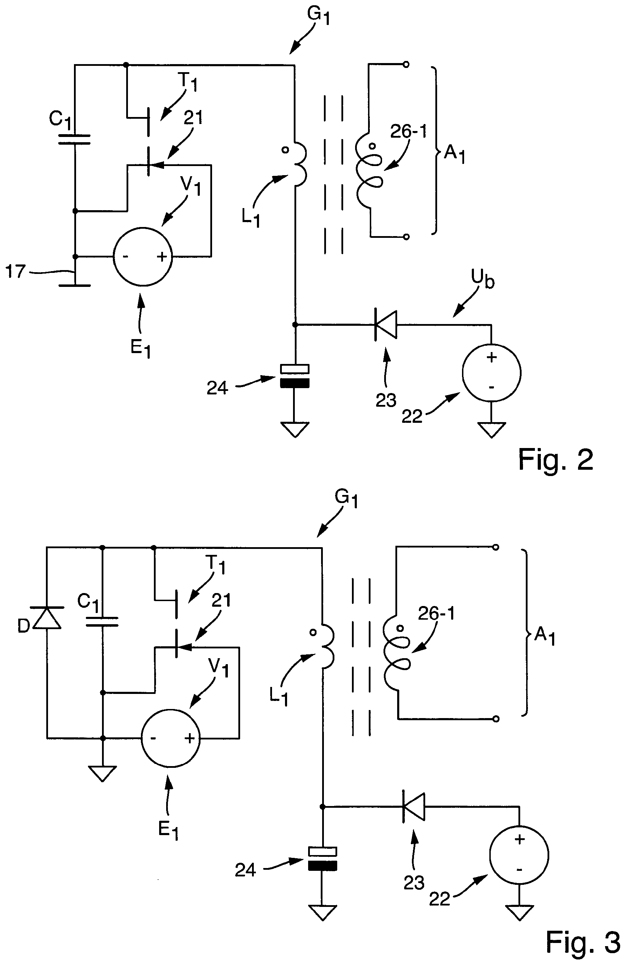

In Figur 2 ist der Impulsgenerator G₁ stellvertretend für alle anderen Impulsgeneratoren G₂ bis Gₙ beschrieben. Die nachfolgende Beschreibung des Impulsgenerators G₁ gilt sowohl hinsichtlich der Struktur seines Aufbaus und seiner Funktion entsprechend für alle anderen Impulsgeneratoren G₂ bis Gₙ.

Der Impulsgenerator G₁ ist vorzugsweise als Sperrwandler aufgebaut. Er umfasst einen Induktor L₁, d.h. beispielsweise eine auf einen Kern gewickelte Spule mit wenigen Windungen oder eine Luftspule, die mit einem Ende an die Betriebsspannung U_{b} und mit ihrem anderen Ende an einen elektronischen Schalter T₁ angeschlossen ist, der den Induktor L₁ wahlweise mit Masse 17 verbindet oder diese Verbindung trennt. Dem elektronischen Schalter T₁ kann ein Schutzkondensator C₁ zugeordnet sein, der parallel zu der Schaltstrecke des elektronischen Schalters geschaltet ist. Ist der elektronische Schalter T₁ ein Transistor beispielsweise ein Feldeffekttransistor, ist die Schaltstrecke, die Drain-Source Strecke.

Dem elektronischen Schalter T₁ ist eine Ansteuerschaltung V₁ zugeordnet, die vorzugsweise zwischen Masse 17 und einer Steuerelektrode 21 des elektronischen Schalters T₁ wirksam ist. die Ansteuerschaltung V₁ kann eine aktive oder eine passive Ansteuerschaltung sein. Sie dient dazu, die gesteuerte Strecke des elektronischen Schalters T₁ durch entsprechende Bereitstellung von Signalen an der Steuerelektrode 21 freizugeben oder zu sperren. Die Ansteuerschaltung 18 weist den Eingang E₁ auf, der mit der Ansteuerschaltung 18 verbunden ist.

Die Betriebsspannung U_{b} wird von einer Gleichspannungsquelle 22, vorzugsweise über eine Entkopplungsdiode 23 bereitgestellt. Dioden 23 der einzelnen Generatoren G₁ bis Gₙ entkoppeln diese voneinander. Die Betriebsspannung U_{B} wird in jedem Impulsgenerator G₁ bis Gₙ vorzugsweise auf einen Pufferkondensator 24 gespeichert.

Der Impulsgenerator G₁ weist einen Ausgang A₁ auf, der durch die Enden einer Spule 26-1 gebildet ist, die transformatorisch mit dem Induktor L₁ koppelt. Im vorliegenden Ausführungsbeispiel sind der Induktor L₁ und die Spule 26-1 gleichsinnig gepolt. Sie können aber auch gegensinnig gepolt sein.

Der Impulsgenerator G₁ nach Figur 2 ist prinzipiell identisch mit dem Impulsgenerator G₁ nach Figur 2 ausgebildet. Die dazu gegebene Beschreibung gilt unter Zugrundelegung gleicher Bezugszeichen entsprechend. Die Besonderheit des Impulsgenerators G₁ nach Figur 3 liegt in der Parallelschaltung einer Diode D zu der Schaltstrecke des elektronischen Schalters T₁ in Sperrrichtung. Sie schützt den Schalter T₁ und verringert den Innenwiderstand des Impulsgeneratorausgangs A₁.

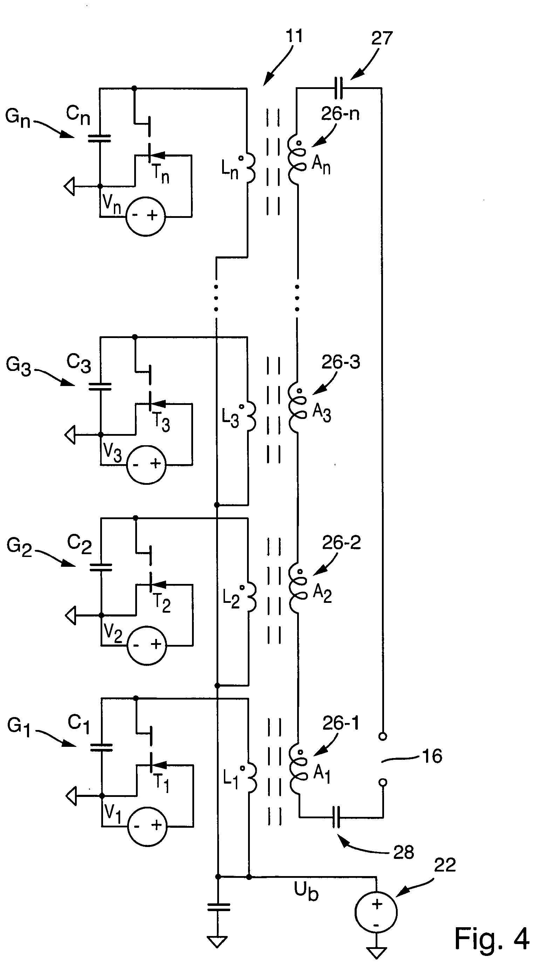

Wie Figur 4 erkennen lässt, sind die Impulsgeneratoren G₁ bis Gₙ gleichspannungsmäßig parallel geschaltet. Die Schalter T₁ ... Tₙ, die Induktoren L₁ ... Lₙ, die Ansteuerschaltungen V₁ ... Vₙ, die Kondensatoren C₁ ... Cₙ und die Spulen 26-1 ... 26-n tragen entsprechende Buchsabenindice, die dem Buchstabenindex des jeweiligen Impulsgenerators G₁ ... Gₙ entsprechen. Ihre Impulsgeneratorausgänge A₁ ... Aₙ, d.h. die Spulen 26-1 bis 26-n sind jedoch gleichsinnig in Reihe geschaltet. In Figur 4 sind die Spulenanfänge der Induktoren L₁ bis Lₙ sowie der Spulen 26-1 bis 26-n jeweils mit einem Punkt gekennzeichnet. Die gleichsinnige Reihenschaltung der Spulen 26-1 bis 26-n bedeutet, dass jeweils ein Spulenanfang mit einem Spulenende der benachbarten Spule verbunden ist. Dies gilt insbesondere, wenn alle Spulenanfänge der Induktoren L₁ bis Lₙ gleichsinnig geschaltet sind, d.h. alle Spulenanfänge jeweils mit dem elektronischen Schalter T₁ bis Tₙ und alle Spulenenden mit der Betriebsspannung U_{B} verbunden sind (oder umgekehrt).

Die Reihenschaltung der Impulsgeneratorausgänge A₁ ... Aₙ kann über einen oder mehrere Koppelkondensatoren 27, 28 mit dem Generatorausgang 16 verbunden sein. Die Koppelkondensatoren 27, 28 können auch an anderen Stellen der Reihenschaltung angeordnet sein oder alternativ auch entfallen. Sind sie vorhanden, beseitigen sie den Gleichanteil des von dem Generator 11 abgegebenen Stroms. Soll ein Gleichanteil des Stroms zugelassen werden, können sie entfallen oder mit einer gesteuerten oder ungesteuerten Überbrückungsschaltung versehen sein.

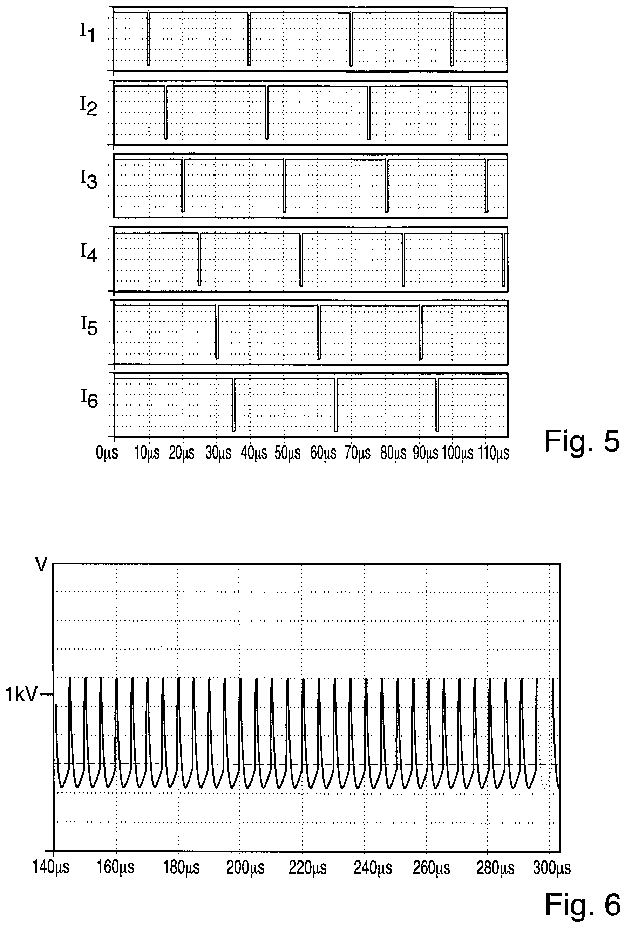

Der insoweit beschriebene Generator 11 arbeitet wie folgt:
Die Steuereinrichtung 18 erzeugt zeitlich koordinierte Ansteuerimpulse für die Impulsgeneratoren G₁ bis Gₙ. Figur 5 veranschaulicht solche Steuerimpulse I₁ bis I₆ für einen Generator 11 mit sechs Impulsgeneratoren G₁ bis G₆ am Beispiel einer Betriebsart (erster Mode) mit gleichmäßigen Zeitabständen. Wie Figur 5 zeigt, kann der Steuerimpuls I₂ für den Impulsgenerator G₂ eine feste Zeitspanne, zum Beispiel 5 µs, nach dem Steuerimpuls I₁ für den Impulsgenerator G₁ abgegeben werden. Entsprechendes gilt jeweils für die anderen Steuerimpulse I₃, I₄, I₅ und I₆. I₃ kann um einen Zeitabstand, z.B. 5 µs, später abgegeben wie I₂, I₄, später als I₃ usw. Im Beispiel nach Figur 5 ist immer ein Zeitabstand von 5 µs angenommen. Die Impulsgeneratoren G₁ bis Gₙ erhalten somit ihre Steuerimpulse I₁ bis Iₙ in Zeitabständen von jeweils 30 µs (sechs Impulsgeneratoren G₁ bis G₆ mal 5µs) . Innerhalb diese Periode erhalten alle anderen Impulsgeneratoren G₁ bis G₆ ihre Steuerimpulse I₁ ... Iₙ jeweils im festen Zeitabstand von hier beispielsweise 5 µs. Entsprechend geben die einzelnen Impulsgeneratoren G₁ bis G₆ ihre Ausgangsimpulse nacheinander im 5µs-Abstand ab. Es ergibt sich die in Figur 6 schematisch veranschaulichte Ausgangsimpulsfolge an dem Ausgang 16 des Generators 11. Die Einzelimpulse der Impulsgeneratoren G₁ bis Gₙ sind weitgehend gleich groß und ergeben zusammen eine Impulsfolge mit einer Grundfrequenz von 333 kHz. Die somit an dem Ausgang 6 anstehende Spannung ist eine asymmetrische hochfrequente Spannung. Mit anderen Zeitabständen zwischen den Steuerimpulsen I₁ ... Iₙ lassen sich andere Grundfrequenzen erzielen.

Die Steuerimpulse I₁ ... In sind Sperrimpulse für die elektronischen Schalter T₁ ... Tₙ. Es können als Steuerimpulse jedoch auch alle anderen Signalformen verwendet werden, die geeignet sind, den Impulsgenerator G₁ ... Gₙ zur Abgabe eines Ausgangsimpulses oder auch einer Folge von Ausgangsimpulsen zu veranlassen.

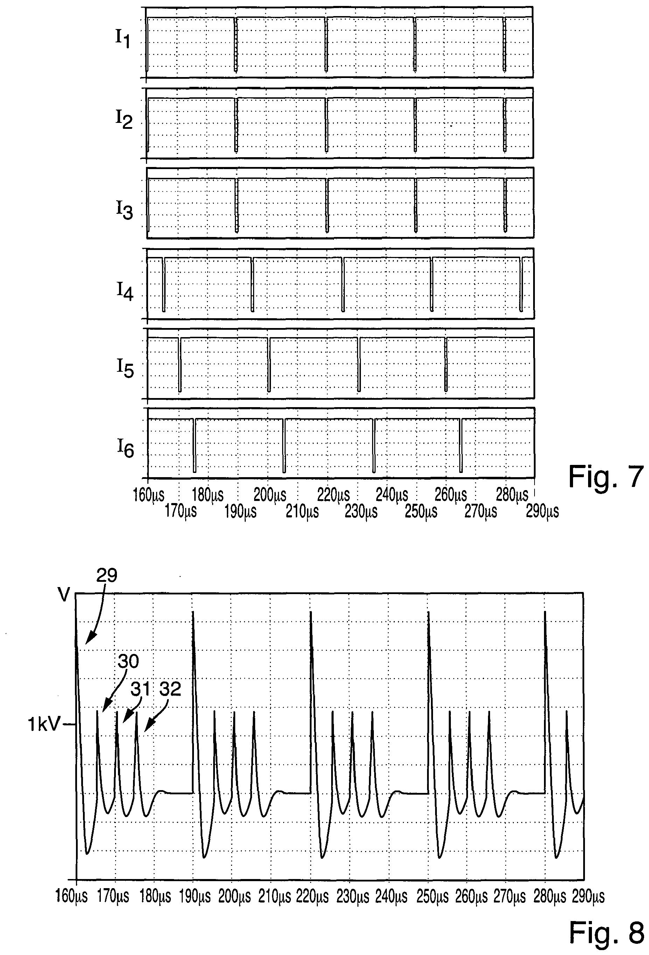

Figur 7 veranschaulicht ein anderes Ansteuermuster bei dem die Steuersignale, d.h. die Steuerimpulse I₁, I₂, I₃ für die Impulsgeneratoren G₁, G₂, G₃ für eine zweite Betriebsart (zweiter Mode) gleichzeitig an die entsprechenden Impulsgeneratoren geliefert werden, während die Steuerimpulse I₄, I₅ und I₆ zeitlich nacheinander an die Impulsgeneratoren G₄, G₅, G₆ gegeben werden. Es entsteht an dem Ausgang 16 des Generators 11 die Impulsfolge nach Figur 8 mit einem großen ersten Ausgangsimpuls 29, der aus der Überlagerung der Ausgangsimpulse der drei gleichzeitig arbeitenden Generatoren G₁, G₂ und G₃ entstanden ist. Die weiteren Ausgangsimpulse 30, 31, 32 sind jeweils einzelne Ausgangsimpulse einzelner Impulsgeneratoren. Nach Abgabe des letzten Ausgangsimpulses 32 kann eine Pause beliebiger Länge, hier bspw. 15 µs folgen.

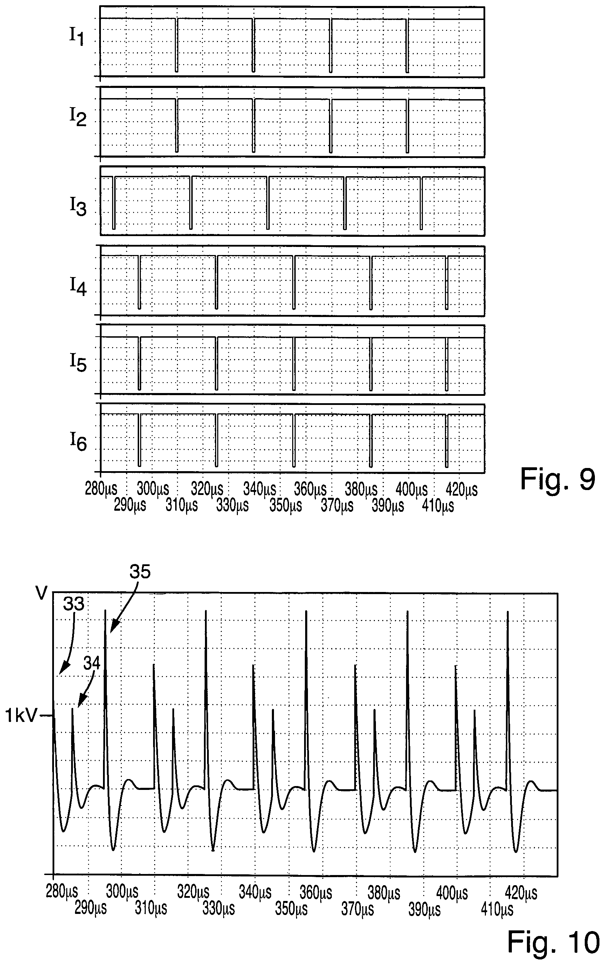

Ein weiteres Beispiel zur Erläuterung der Flexibilität der Signalgestaltung ergibt sich aus den Figuren 9 und 20, die eine dritte Betriebsart (dritter Mode) veranschaulichen. Gemäß Figur 9 werden die Generatoren G₁ und G₂ zeitlich synchron mit Steuerimpulsen I₁ und I₂ versorgt und erzeugen somit einen verdoppelten Ausgangsimpuls 33. Darauf folgt der Steuerimpuls I₃ für den Generator G₃, der einen einfachen Ausgangsimpuls 34 erzeugt. Die Impulsgeneratoren G₄, G₅ und G₆ erhalten ihre Steuerimpulse I₄, I₅, I₆ wiederum gleichzeitig und erzeugen somit den dreifache Ausgangsimpuls 35.

Wie ersichtlich, sind durch das Timing der Steuerimpulse I₁ bis I₆ verschiedene Ausgangsspannungsformen erzielbar, die mit einem Resonanzgenerator nicht erzeugbar wären. Damit eröffnet das vorgestellte Schaltungsprinzip die Möglichkeit der Erzeugung von Spannungsformen mit physiologischen Wirkungen, die mit bisherigen Generatoren nicht erreichbar waren. Außer den hier explizit veranschaulichten Modes sind weitere Modes erzeugbar, indem das Timing der Steuerimpulse I₁ ... Iₙ und somit der Ausgangsimpulse A₁ ... Aₙ der Impulsgeneratoren G₁ ... Gₙ und/oder die Anzahl der Impulsgeneratoren G₁ ... Gₙ und/oder die Polung der Spulen 26-1 ... 26-n variiert wird.

Ein erfindungsgemäßer Generator 11 weist eine Anzahl von Impulsgeneratoren G₁ bis Gₙ auf, die von einer Steuereinrichtung 18 zeitlich flexibel einzeln angesteuert werden. Die zur Versorgung eines chirurgischen Instruments 12 erforderliche HF-Spannung wird dadurch aus Einzelimpulsen zusammengesetzt. Gleiches gilt für den an der Elektrode 14 des Instruments 12 fließenden Strom. Durch den Wegfall von Resonanzeffekten in den Impulsgeneratoren G₁ bis Gₙ und den Wegfall der Energiespeicherung in einem schwingungsfähigen System (System zweiter Ordnung) erhält der Nutzer ein erhöhtes Maß an Kontrolle über die Kurvenform der an das Instrument 12 gelieferten Spannung und des zu dem Instrument 12 fließenden Stroms.

### Bezugszeichen:

- 11: Generator
- 12: Instrument
- 13: Handgriff
- 14: Elektrode
- 15: Neutralelektrode
- 16: Ausgang
- G₁ ... Gₙ: Impulsgeneratoren
- U_{b}: Betriebsspannung
- 17: Masse
- A₁ ... Aₙ: Impulsgeneratorausgänge
- 18: Steuereinrichtung
- L₁ ... Lₙ: Induktoren
- E₁ ... E₂: Steuersignaleingänge der Impulsgeneratoren G₁ ... Gₙ
- I₁ ... Iₙ: Steuerimpulse für die Impulsgeneratoren G₁ ... Gₙ
- S: Steuereingang
- T₁ ... Tₙ: elektronischer Schalter
- C₁ ... Cₙ: Schutzkondensatoren
- V₁ ... Vₙ: Ansteuerschaltungen
- 21: Steuerelektrode
- 22: Gleichspannungsquelle
- 23: Entkopplungsdiode
- 24: Pufferkondensator
- 26-1...26-n: Spulen
- 27, 28: Koppelkondensatoren
- 29 ... 35: Ausgangsimpulse

## Patentansprüche

1. Generator (11):
Mit einem Ausgang (16), an den ein medizinisches Instrument (12) anschließbar ist,
mit einer Anzahl von Impulsgeneratoren (G₁ ... Gₙ), die jeweils einen Steuereingang (E₁ ... Eₙ) und einen Impulsgeneratorausgang (26-1 ... 26-n) aufweisen,
mit einer Steuereinrichtung (18), die mit den Steuereingängen (E₁ ... Eₙ) verbunden ist, um an den jeweiligen Impulsgenerator (G₁ ... Gₙ) einen Ansteuerimpuls (I₁ ... Iₙ) zu geben, um den Impulsgenerator (G₁ ... Gₙ) zur Abgabe eines Ausgangsimpulses (29 ... 35) zu veranlassen,
wobei die Impulsgeneratorausgänge (A₁ ... Aₙ) aller Impulsgeneratoren (G₁ ... Gₙ) mit dem Ausgang (16) des Generators (11) verbunden sind.

2. Generator nach Anspruch 1, **dadurch gekennzeichnet, dass** alle Impulsgeneratoren (G₁ ... Gₙ) untereinander gleich ausgebildet sind.

3. Generator nach einem der vorstehenden Ansprüche,
**dadurch gekennzeichnet, dass** jeder Impulsgenerator (G₁ ... Gₙ) darauf eingerichtet ist, in Reaktion auf den Empfang eines Steuerimpulses (I₁ ... Iₙ) an seinem Steuereingang (E₁ ... Eₙ) an seinem Impulsgeneratorausgang (A₁ ... Aₙ) einen Ausgangsimpuls (29 ... 35) abzugeben.

4. Generator nach einem der vorstehenden Ansprüche,
**dadurch gekennzeichnet, dass** die Steuereinrichtung (18) dazu eingerichtet ist, Steuerimpulse (I₁ ... Iₙ) an zumindest zwei der Impulsgeneratoren (G₁ ... Gₙ) zeitlich aufeinanderfolgend abzugeben.

5. Generator nach einem der vorstehenden Ansprüche,
**dadurch gekennzeichnet, dass** die Steuereinrichtung (18) darauf eingerichtet ist, Steuerimpulse (I₁ ... Iₙ) an zumindest zwei der Impulsgeneratoren (G₁ ... Gₙ) gleichzeitig abzugeben.

6. Generator nach einem der vorstehenden Ansprüche,
**dadurch gekennzeichnet, dass** die Steuereinrichtung (18) dazu eingerichtet ist, Steuerimpulse (I₁ ... Iₙ) nach verschiedenen zeitlichen Mustern abzugeben.

7. Generator nach einem der vorstehenden Ansprüche,
**dadurch gekennzeichnet, dass** die Impulsgeneratorausgänge (A₁ ... Aₙ) elektrisch in Reihe geschaltet sind.

8. Generator nach einem der vorstehenden Ansprüche,
**dadurch gekennzeichnet, dass** jeder Impulsgenerator (G₁ ... Gₙ) einen Energiespeicher aufweist.

9. Generator nach Anspruch 8, **dadurch gekennzeichnet, dass** der Energiespeicher ein Induktor (L₁ ... Lₙ) ist.

10. Generator nach Anspruch 9, **dadurch gekennzeichnet, dass** der Impulsgeneratorausgang (A₁ ... Aₙ) eine mit dem Induktor (L₁ ... Lₙ) transformatorisch koppelnde Spule (26-1 ... 26-n) ist.

11. Generator nach einem der vorstehenden Ansprüche,
**dadurch gekennzeichnet, dass** der Impulsgenerator (G₁ ... Gₙ) ein Sperrwandler ist.

12. Verfahren zur Erzeugung einer elektrischen Spannung zur Versorgung eines elektrochirurgischen Instruments (12) mit einer Folge von Stromimpulsen (29 ... 35), wobei bei dem Verfahren
die Folge von Stromimpulsen (29 ... 35) mittels mehreres ausgangsseitig miteinander verbundener Impulsgeneratoren (G₁ ... Gₙ) erzeugt wird.

13. Verfahren nach Anspruch 12, **dadurch gekennzeichnet, dass** wenigstens zwei der Impulsgeneratoren (G₁ ... Gₙ) dazu veranlasst werden, gleichzeitig Stromimpulse (29, 33, 35) abzugeben.

14. Verfahren nach Anspruch 12 oder 13, **dadurch gekennzeichnet, dass** wenigstens zwei der Impulsgeneratoren (G₁ ... Gₙ) dazu veranlasst werden, Stromimpulse (30, 31, 32) zeitlich nacheinander abzugeben.
